(19) Europäisches Patentamt

European Patent Office

Office européen des brevets

(11) **EP 0 760 650 B1**

(12) **EUROPEAN PATENT SPECIFICATION**

(45) Date of publication and mention
of the grant of the patent:
**31.07.2002   Bulletin 2002/31**

(51) Int Cl.$^7$: **A61K 31/14**, A61K 31/23,
A61K 9/107, A61P 31/00

(21) Application number: **95920506.3**

(22) Date of filing: **18.05.1995**

(86) International application number:
**PCT/US95/06236**

(87) International publication number:
**WO 95/31966 (30.11.1995 Gazette 1995/51)**

(54) **ANTIMICROBIAL OIL-IN-WATER EMULSIONS**

ANTIMIKROBIELLE ÖL-IN-WASSER-EMULSIONEN

EMULSIONS HUILE-DANS-EAU ANTIMICROBIENNES

(84) Designated Contracting States:
**BE CH DE ES FR GB IE IT LI NL SE**

(30) Priority: **20.05.1994   US 246868**
**13.10.1994   US 322827**
**26.10.1994   US 329730**

(43) Date of publication of application:
**12.03.1997   Bulletin 1997/11**

(73) Proprietor: **NOVAVAX, INC.**
**Rockville, MD 20852 (US)**

(72) Inventor: **WRIGHT, D.Craig**
**Gaithersburg, MD 20878 (US)**

(74) Representative: **Duffy, Assumpta Dympna et al**
**F. R. Kelly & Co.,**
**27 Clyde Road,**
**Ballsbridge**
**Dublin 4 (IE)**

(56) References cited:
**EP-A- 0 550 960          WO-A-89/06124**
**US-A- 5 362 494**

**Description**

**Background of the Invention**

The present invention relates to an antimicrobial lipid-containing oil-in-water emulsion which inactivates infectious pathogens upon contact.

It is known that if a water-immiscible liquid phase is mixed into an aqueous phase by mechanical agitation, for example, by means of an ultra-disperser, the stability of the resulting oil-in-water dispersion most frequently requires the addition of an emulsifying agent, the molecules of which are adsorbed onto the surface of the oil droplets to form a kind of continuous membrane which prevents direct contact between two adjacent droplets. The drops of oil can further contain substances soluble in an organic medium, such as a sterol.

In addition to discrete oil droplets dispersed in an aqueous phase, oil-in-water emulsions can also contain other lipid structures, such as small lipid vesicles (i.e., lipid spheres which often consist of several substantially concentric lipid bilayers separated from each other by layers of aqueous phase), micelles (e.g., amphiphile molecules in small clusters of 50-200 molecules arranged so that the polar head groups face outward toward the aqueous phase and the apolar tails are sequestered inward away from the aqueous phase), or lamellar phases (lipid dispersions in which each particle consists of parallel glycerol ester bilayers separated by thin films of water). These lipid structures are formed as a result of hydrophobic forces which drive apolar residues (i.e., long hydrocarbon chains) away from water.

The portals of entry of pathogenic bacteria, viruses or fungi are predominantly the skin and mucus membranes, and upper and lower respiratory tracts. The first step in any infection is attachment or colonization on skin or mucus membranes with subsequent invasion and dissemination of the infectious pathogen. Accordingly, an object of the present invention is to provide an antimicrobial emulsion which inactivates infectious pathogens on contact by disrupting their membrane structures.

**Summary of the Invention**

The present invention provides a stable antimicrobial oil-in-water emulsion for inactivating infectious pathogens upon contact. The emulsion comprises positively charged droplets of a lipid-containing oily "discontinuous phase" dispersed in an aqueous "continuous phase". It is believed that the oily discontinuous phase inactivates infectious pathogens by disrupting their membrane structure. The emulsion has microbicidal activity against a broad spectrum of bacteria, viruses and yeasts.

The antimicrobial emulsion of the present invention consists primarily of positively charged droplets of an oily discontinuous phase dispersed in an aqueous continuous phase, such as water. The discontinuous phase contains glycerol monooleate (GMO) and a cationic halogen-containing compound having a $C_{12}$-$C_{16}$ chain as defined in claim 1 as a positive charge producing agent. The droplets can further contain a sterol, such as cholesterol or phytosterol. The droplets appear to bind to negatively charged proteins contained in bacterial, viral, or fungal membranes, thereby disrupting the membrane structure and irradiating the pathogen.

Antimicrobial emulsions of the present invention are non-toxic and safe, for example, when swallowed, inhaled, or applied to the skin. This result is unexpected since many cationic halogen-containing compounds having a $C_{12}$-$C_{16}$ chain are extremely toxic if administered alone. For example, cetylpyridinium chloride (CPC), the preferred cationic halogen-containing compound of the invention, causes severe irritation and damage to tissues of the upper respiratory tract, mucous membranes and skin. However, when administered in the form of an emulsion of the invention, no such adverse effects occur. Furthermore, the emulsions of the invention are stable when heated or exposed to significant levels of acid and base.

The positive charge of the oily discontinuous phase is provided by a cationic halogen-containing compound having a $C_{12}$-$C_{16}$ chain, which is selected from the group consisting of cetylpyridinium chloride (CPC), cetylpyridinium bromide (CPB), cetyltrimethylammonium bromide (CTAB), and cetyldimethyethylammonium bromide (CDEAB). Other cationic halogen-containing compounds having a $C_{12}$-$C_{16}$ chain which can be used are cetyltrimethylammonium chloride, cetylbenzyldimethylammonium chloride, cetyltributylphosphonium bromide, dodecyltrimethylammonium bromide, and tetradecyltrimethylammonium bromide.

The oily discontinuous phase can further contain at least one sterol selected from the group consisting of cholesterol, cholesterol derivatives, hydrocortisone, phytosterol, and mixtures thereof. The term "cholesterol derivatives," as used herein, includes but is not limited to sulfate and phosphate derivatives of cholesterol. Preferred sterols include phytosterols, such as soya sterol.

Oils useful in forming antimicrobial oil-in-water emulsions of the present invention include a broad spectrum of water-immiscible materials, such as soybean oil, avocado oil, squalene oil, sesame oil, olive oil, canola oil, corn oil, rapeseed oil, safflower oil, sunflower oil, fish oils, flavor oils, water insoluble vitamins, and mixtures thereof. In a preferred embodiment of the invention, the oil is a scented or flavored oil such as peppermint oil.

In another embodiment of the invention, at least a portion of the emulsion may be in the form of lipid structures including, but not limited to, unilamellar, multilamellar, and paucilamellar lipid vesicles, micelles, and lamellar phases.

The antimicrobial emulsions of the present invention can be used, for example, in pharmaceutical preparations (e.g., creams, solutions and suspensions) to inhibit the growth of a wide variety of infectious pathogens, including bacteria, viruses, and fungi. Accordingly, the present invention also provides an antimicrobial preparation suitable for pharmaceutical administration made up of an antimicrobial emulsion of the invention and a pharmaceutically acceptable carrier. The preparation can be applied topically to skin surface areas, mucus membranes, or oral surfaces, for example, as a cream, gel, spray, mouthwash, or deodorant to treat or prevent bacterial infections such as *Propionibacterium acnes, Neisseria gonorrhea, Streptococcus, and Staphylococcus epidermidis.* Alternatively, the preparation can be administered internally by, for example, oral or intravenous administration. Such systemic administration of the preparation can be used, for example, to inactivate pathogenic microorganisms, e.g., bacteria such as *Helicobacter pylori,* and viruses, particularly envelope viruses. In a preferred embodiment of the invention, the preparation is administered orally to an individual to treat infection by HIV.

## Detailed Description of the Invention

The present invention relates to a stable antimicrobial oil-in-water emulsion made of positively charged droplets of an oily discontinuous phase dispersed in an aqueous continuous phase.

The term "antimicrobial," as used herein, means having the ability to inactivate infectious pathogens. The term "inactivate", as used herein, includes but is not limited to, killing or inhibiting growth of the organism. The term "infectious pathogen", as used herein, includes, but is not limited to, fungi, viruses, bacteria, and parasites. The antimicrobial emulsion of the present invention inactivates a wide variety of infectious pathogens. It appears that inactivation is achieved by disruption of the membrane structure of the pathogen by the components of the oily discontinuous phase.

The term "emulsion," as used herein, includes both classic oil-in-water dispersions or droplets, as well as other lipid structures which can form as a result of hydrophobic forces which drive apolar residues (i.e., long hydrocarbon chains) away from water and drive polar head groups toward water, when a water immiscible oily phase is mixed with an aqueous phase. These other lipid structures include, but are not limited to, unilamellar, paucilamellar, and multilamellar lipid vesicles, micelles, and lamellar or hexagonal phases. These other lipid structures also contain glycerol monooleate (GMO) as the primary emulsifier, and a dodecyl to hexadecyl cationic halogen-containing compound as a positive charge producing agent. These other lipid structures can also further include at least one sterol, preferably a phytosterol. The term "primary emulsifier", as used herein, refers to the emulsifier which constitutes the greatest proportion by weight of any single emulsifier contained in the oily discontinuous phase.

Antimicrobial oil-in-water emulsions of the present invention can be formed using classic emulsion forming techniques which are well known in the art. In brief, the lipid-oil phase is mixed with the aqueous phase under relatively high shear forces to obtain an oil-in-water emulsion containing oil droplets which are approximately 1 micron in diameter. More particularly, a positively charged lipid-containing oily discontinuous phase is formed by blending (a) an oil carrier, (b) GMO; and (c) a cationic halogen-containing compound having a $C_{12}$-$C_{16}$ chain as defined in claim 1, along with any other compatible glycerol esters or emulsifiers, such as Polysorbate 60, and any sterols or other lipophilic materials to be incorporated into the lipid-oil phase.

Once the lipid-oil phase is formed, it is heated and blended with an aqueous phase (e.g., water, saline, or any other aqueous solution which can hydrate the lipids) on a volume to volume basis ranging from about 1:4 to 1:2, preferably about 1:3 lipid-oil phase to aqueous phase. The lipid-oil and aqueous phases can be blended using any apparatus capable of producing the high shear mixing forces, including, for example, a french press, a Novamix™ lipid vesicle maker (IGI Inc., Buena NJ.), a syringe mixer, or, alternatively by hand using two syringes as described in U.S. Patents Nos. 4,895,452 and 4,911,928.

Antimicrobial oil-in-water emulsions of the present invention provide the advantage of being stable in the presence of heat, acid, or base. For example, as shown below in Example 7, emulsions of the invention are not significantly altered or broken down when boiled or exposed to IN nitric acid or IN sodium hydroxide. This stability makes the emulsions suitable for pharmaceutical administration, even internal administration.

Antimicrobial oil-in-water emulsions of the present invention can be used to inactivate a variety of infectious pathogens upon contact. As described in the examples below, microbes which are inactivated by the present invention include a wide variety of bacteria, fungi and viruses. More specifically, for inactivation of bacteria which colonize in oral or oropharyngeal areas, such as *Streptococcus pneumoniae,* Group A beta-hemolytic *Streptococcus, Haemophilus influenzae,* and *Neisseria meningitidis,* the presently disclosed emulsions can be applied topically, as a spray or mouthwash. For bacteria which colonize in gastrointestinal areas, such as *Helicobacter pylori,* the emulsions can be administered orally, for example, as a pill or a liquid. For bacteria and fungi which colonize in the vagina, such as *Neisseria gonorrhoeae, Gardnerella vaginalis,* Group B *Streptococcus,* and *Candida albicans,* the emulsions can be applied topically as a cream, gel, or suppository. The presently disclosed emulsions can also be used for dermatological application as a cream or gel to inactivate or prevent infection secondary to *Propionibacterium acnes, Staphylococcus aureus, Staphylococcus epidermidis,* and Group B *Streptococcus.* In a preferred embodiment of the invention, antimicrobial emulsions of the present invention are used to prevent infection by gram positive bacteria.

Antimicrobial oil-in-water emulsions of the present invention can also be used to inactivate a variety of viruses, particularly enveloped viruses upon contact. For example, the presently disclosed emulsions can be used for oropharyngeal, oral, veneral, dermatological, or internal application to inactivate or prevent infection secondary to viruses including *Herpesviridae* (e.g., herpes simplex types 1 and 2, Epstein-Barr virus, cytomegalovirus, varicella virus, and human herpes virus type 6), *Togaviridae* (e.g., Rubella virus), *Flaviviridae,* (e.g., Yellow Fever virus), *Coronaviridae (e. g.,* Corona viruses), *Rhabdoviridae (e.g.,* Rabies virus), *Filoviridae (e.g.,* Marburg virus), *Paramyxoviridae (e.g.,* Measles virus), *Orthomyxoviridae* (*e.g.,* Influenza viruses), *Bunyaviridae (e.g.,* California encephalitis virus), *Arenaviridae (e.g.,* Lymphocytic choriomeningitis virus), *Retroviridae (e.g., HIV-1)*, and *Hepadnavirida* (e.g., Hepatitis B). The emulsions of the invention can also be used to inactivate viruses in vitro, for example, to disinfect contaminated blood products. In a preferred embodiment of the invention, the oil-in-water emulsions are used to inactivate HIV.

The present invention also provides an antimicrobial preparation suitable for pharmaceutical administration consisting of the antimicrobial emulsion of the present invention and a pharmaceutically acceptable carrier. The term "pharmaceutically acceptable carrier," as used herein, refers to any physiologically compatible carrier for use in pharmaceutical administration. Use of such media and agents for pharmaceutically active substances is well known in the art. Except insofar as any conventional media or agent is incompatible with the emulsions of the present invention, use thereof in a pharmaceutical preparation is contemplated.

The anti microbial emulsions of the present invention can be used in methods for inhibiting the growth of an infectious pathogen by topical, or systemic administration of the antimicrobial emulsion of the present invention, preferably in the form of a pharmaceutical preparation. The term "topical," as used herein, includes application to mucous membranes, oral surfaces, skin, inner ear surfaces, or the surfaces of any bodily orifice, such as the vagina or rectum. The term "systemic", as used herein, includes any form of internal administration, including but not limited to, oral and intravenous administration.

The following examples will illustrate the efficacy of the invention.

## EXAMPLES

## Example 1 - Preparation of GMO and GMS* Oil-in-Water Emulsions

In this Example, a series of positively and negatively charged lipid-containing oil-in-water emulsions having either GMO or GMS as the primary emulsifier were formed. Selected emulsions were also characterized with regard to purity, pH and size of oil droplets.

Table 1 shows the amount of each chemical component used to form the lipid-oil phase of several charged GMO and GMS oil-in-water emulsions having glycerol monooleate (GMO) or glycerol monostearate (GMS) as the primary emulsifiers, and cetylpyridinium chloride (CPC), cetylpyridinium bromide (CPB), cetyltrimethylammonium bromide (CTAB), or dimethyldioctadecylammonium bromide (DDDAB) as positive charge producing agents, or oleic acid as a negative charge producing agent.

TABLE 1

| | GMO/ CPC | GMO/ CPB | GMO/ CTAB | GMS/ DDDAB | GMO/ OLEIC | GMS/ CPC |
|---|---|---|---|---|---|---|
| GMO | 3.43 g | 3.43 g | 3.43 g | ------ | 3.43 g | ------ |
| GMS | ------ | ------ | ------ | 3.43 g | ------ | 3.43 g |
| Soya Sterol or cholesterol | 0.96 g | 0.96 g | 0.96 g | 0.96 g | 0.96 g | 0.96 g |
| Tween 60 | 0.84 g | 0.84 g | 0.84 g | 0.84 g | 0.84 g | 0.84 g |
| Soybean Oil | 11 g | 11 g | 11 g | 11 g | 11 g | 11 g |
| CPC | 130 mg | ------ | ------ | ------ | ------ | 130 mg |
| CPB | ------ | 150 mg | ------ | ------ | ------ | ------ |
| CTAB | ------ | ------ | 140mg | ------ | ------ | ------ |
| DDDAB | ------ | ------ | ------ | 240 mg | ------ | ------ |
| Oleic Acid | ------ | ------ | ------ | ------ | 108 mg | ------ |

*for reference only

To prepare the charged emulsions shown above in Table 1, a lipid-oil phase containing approximately 12 to 21% by weight GMO or GMS, 0.8 to 0.9% by weight cationic halogen-containing compound (or 0.7% by weight oleic acid), 67 to 80% by weight carrier oil such as soybean oil, 3 to 5% by weight Tween 60 (Polyoxyethylene 20 sorbitan monostearate), and 3 to 6% by weight soya sterol was heated for approximately one hour at 86°C. The lipid-oil phase was then blended with an aqueous phase containing water at 65°C using a 5 ml syringe machine as described in US Patent No. 4,895,452 on a volume to volume basis of 13 parts lipid-oil to 37 parts water.

Table 2 shows the pH of the emulsions shown in Table 1. Also shown is the size of the lipid-oil droplets of the emulsions measured on a Coulter LS 130 Laser sizing instrument equipped with a circulating waterbath.

TABLE 2

| Chemical Components of Emulsion | Charge | pH | Mean Coulter Size in Microns | Mean Coulter Range in Microns |
|---|---|---|---|---|
| GMO/CPC | Positive | 3.72 | 1.049 | 0.720-1.401 |
| GMO/CPB | Positive | 4.31 | 0.891 | 0.680-1.124 |
| GMO/CTAB | Positive | 4.82 | 1.143 | 0.647-1.358 |
| GMS/DDDAB | Positive | 5.86 | 1.080 | 0.694-1.532 |
| GMO/Oleic acid | Negative | 4.45 | 1.078 | 0.738-1.448 |
| GMS/CPC | Positive | 3.72 | 1.047 | 0.677-1.497 |

## Example 2 - Preparation of 10N GMO Oil-in-Water Emulsions

In this Example, a series of positively charged oil-in-water emulsions were prepared having GMO as the primary emulsifier and a 10 fold higher percentage (10N) of cationic halogen-containing compound (cetylpyridinium chloride (CPC), cetylpyridinium bromide (CPB), cetyltrimethylammonium bromide (CTAB), cetyldimethyethylammonium bromide (CDEAB), and benzalkonium chloride (BAC)), as compared with Example 1, as a positive charge producing agent.

Table 3 shows the amount of each chemical component used to form the lipid-oil phase of the positively charged 10N GMO emulsions.

TABLE 3

| Positively Charged 10N Emulsions | | | | | |
|---|---|---|---|---|---|
| Chemical Components of Emulsion | GMO/ CPC | GMO/ CPB | GMO/ CTAB | GMO/ CDEAB | GMO/ BAC |
| GMO | 3.43 g | 3.43 g | 3.43 g | 3.43g | 3.43 g |
| Soya Sterol or cholesterol | 0.96 g | 0.96 g | 0.96 g | 0.96 g | 0.96 g |
| Tween 60 | 0.84 g | 0.84 g | 0.84 g | 0.84 g | 0.84 g |
| Soybean Oil | 11 g | 11 g | 11 g | 11 g | 11 g |
| CPC | 1.3 g | ----- | ----- | ----- | ----- |
| CPB | ----- | 1.5g | ----- | ----- | ----- |
| CTAB | ----- | ----- | 1.4 g | ----- | ----- |
| CDEAB | ----- | ----- | ----- | 1.3 g | ----- |
| BAC | ----- | ----- | ----- | ----- | 1.3 g |

To prepare the 10N emulsions shown above in Table 3, a lipid-oil phase containing approximately 19% by weight GMO, 62% by weight carrier oil such as soybean oil, 4.7% by weight Tween 60 (Polyoxyethylene 20 sorbitan monostearate), 5.4% by weight soya sterol, and 7.4 to 8.4% by weight cationic detergent was heated for approximately one hour at 86°C. The lipid-oil phase was then blended with an aqueous phase containing water at 65°C using a 5 ml syringe machine (as described in U.S. Patent No. 4,895,452) on a volume to volume basis of 13 parts lipid-oil to 37 parts water.

## Example 3 - Microbicidal Activity of GMO and GMS Oil-in-Water Emulsions Against *Staphylococcus aureus* type 8

In this Example, the microbicidal activities of each of the GMO and GMS emulsions shown in Table 1 were compared. For this purpose, the ability of each of the emulsions to kill *Staphylococcus aureus* type 8 was tested as follows:

Bacteria were innoculated into liquid media (Trypticase soy broth or Schaedler's broth) and incubated at 37°C for 1-7 hours. An optical density at a wavelength of 650 nm and a quantitative culture were then performed on each broth culture. One milliliter of each bacterial broth was then mixed with one milliliter of emulsion, or water as a control, for 10 or 30 minutes. Quantitative cultures were then performed in duplicate on each mixture. Plates containing bacteria were incubated at 37°C for 18 hours and then counted.

The percentage of bacteria killed was then determined by the following equation.

$$\% \; kill = \frac{(A-B)}{A} \; X \; 100$$

A = The total number of bacteria innoculated
B = The total number counted after mixing with an emulsion
Table 4 lists the percentages of *Staphylococcus aureus* type 8 killed by each emulsion or water after a 10 or 30 minute incubation with $2 \times 10^7$ bacteria.

TABLE 4

| Chemical Components of Preparation | % Inactivate After a 10 Minute Incubation | % Inactivate After a 30 Minute Incubation |
|---|---|---|
| GMO/CPC | 100 | 100 |
| GMO/CPB | 100 | 100 |
| GMO/CTAB | 99.99 | 99.99 |
| GMO/DDDAB | 65 | 0 |
| GMO/Oleic acid | 0 | 0 |
| GMS/CPC | 65 | 0 |
| Control alone | 50 | 0 |

From Table 4 one can see that the positively charged glycerol monooleate (GMO) emulsions inactivate *Staphylococcus aureus* type 8 while the positively charged glycerol monostearate (GMS/CPC) emulsion fails to inactivate. In addition, only the cationic compounds having a $C_{12}$-$C_{16}$ chain containing either chloride or bromide, i.e., CPC, CPB or CTAB, and not the dioctadecyl cationic compound (DDDAB) or oleic acid (having a negative charge), were associated with significant microbicidal activity. The inactivation of this bacterium, *Staphylococcus aureus,* is therefore more effective using GMO emulsions containing a chloride or bromide containing cationic compound having a $C_{12}$-$C_{16}$ chain.
In order to determine the effect of varying the concentration of the charge producing agent in the emulsions of the invention, GMO emulsions containing different concentrations of CPC were also tested for antimicrobicidal activity against *Staphylococcus aureus* type 8. Table 5 shows percentages of *Staphylococcus aureus* type 8 killed after a 10 or 30 minute incubation of the GMO emulsions with $10^7$ bacteria.

TABLE 5

| Chemical Components of Emulsion | Initial Innoculum of Staphylococcus Aureus Type 8 | % Inactivate After a 10 Minute Incubation | % Inactivate After a 30 Minute Incubation |
|---|---|---|---|
| GMO/CPC | 10,000,000 CFU [1.8 mg/ml emulsion] | 100 | 100 |
| GMO/CPC | 10,000,000 CFU [0.9 mg/ml emulsion] | 99.5 | 100 |
| GMO/CPC | 10,000,000 CFU [0.45 mg/ml emulsion] | 54 | 99.5 |
| GMO/CPC | 10,000,000 CFU [0.23 mg/ml emulsion] | 39 | 32 |
| GMO/CPC | 10,000,000 CFU [0.028 mg/ml emulsion] | 0 | 0 |

TABLE 5   (continued)

| Chemical Components of Emulsion | Initial Innoculum of Staphylococcus Aureus Type 8 | % Inactivate After a 10 Minute Incubation | % Inactivate After a 30 Minute Incubation |
|---|---|---|---|
| GMO | 10,000,000 CFU | 0 | 0 |
| GMO/Oleic acid | 10,000,000 CFU | 0 | 0 |
| Water alone | 10,000,000 CFU | 10 | 0 |

Table 5 demonstrates that *Staphylococcus aureus* type 8 bacteria were sensitive to the microbicidal action of the GMO/CPC emulsion at CPC concentrations of greater than 0.23 mg/ml of emulsion.

**Example 4 - Microbicidal Activity of GMO Oil-in-Water Emulsions Against Bacteria and Yeast**

In this Example, the ability of the GMO/CPC emulsion formed in Example 1 and shown in Table 1 to kill a variety of bacteria and yeast was tested.
The assay described above in Example 3 was used to measure the microbicidal activity of the GMO/CPC emulsion against a number of bacteria and fungi, except for the following changes:

Yeast were innoculated into Sabouraud's broth and incubated at 30°C for 6 hours, mixed with the GMO/CPC emulsion for 10 or 30 minutes, plated and incubated at 37 C for 18 hours before counting.

*P. acnes* was grown anaerobically in Schaedler's broth for 24 hours, mixed with emulsion for 10 or 30 minutes, plated on Trypticase soy agar plates with 5% sheep blood and incubated anaerobically for 72 hours prior to counting.

*G. vaginalis* was plated on Trypticase soy agar plates with 5% sheep blood and incubated in 5% $CO_2$ at 37°C for 72 hours. Colonies were swabbed from plates and innoculated into Schaedler's broth at the density of a 0.5 McFarland standard. This broth/bacterial mixture was then incubated with the emulsion for 10 or 30 minutes and then plated on Trypticase soy agar plates with 5% sheep blood. Plates were incubated for 72 hours in 5% $CO_2$ at 37°C before counting colonies.

Table 6 lists the percentage of gram positive bacteria killed after a 10 or 30 minute incubation of the GMO/CPC emulsion with between $10^5$ and $10^8$ bacteria. The listed bacteria can generally be categorized as follows: (a) those which colonize on the skin which include *Staphylococcus aureus* (type 8), *Staphylococcus aureus* (type 5), *Staphylococcus epidermidis* (strain 977), Group B *Streptococcus* (capsular type III), Group A *Streptococcus* (beta-hemolytic), and *Propionibacterium acneus;* (b) those which colonize in the oropharynx which include Group A *Streptococcus* (beta-hemolytic), *Streptococcus pneumoniae* (type 5), and *Streptococcus mutans;* and (c) those which colonize or infect the vagina which include *Gardnerella vaginalis* and Group B *Streptococcus* (capsular type III).

TABLE 6

| Gram Positive Bacteria | Innoculum (CPU)• | % Inactivate after a 10 Minute Incubation | % Inactivate after a 30 Minute Incubation |
|---|---|---|---|
| *Staphylococcus aureus* (type 8/bactermic isolate) | $2 \times 10^7$ | 99.99 | 99.99 |
| *Staphylococcus aureus* (type 5 bacteremic isolate) | $9 \times 10^6$ | 100 | 99.99 |
| *Staphylococcus epidermidis* (strain 977) | $8 \times 10^5$ | 100 | 100 |
| Group B *Streptococcus* (capsular type III) | $2.9 \times 10^7$ | 99.99 | 100 |
| Group A beta-hemolytic (type 1) *Streptococcus* (ATCC 12344) | $3.3 \times 10^7$ | 99.99 | 99.99 |

• CPU = colony forming units

TABLE 6   (continued)

| Gram Positive Bacteria | Innoculum (CPU)• | % Inactivate after a 10 Minute Incubation | % Inactivate after a 30 Minute Incubation |
|---|---|---|---|
| *Listeria monocytogenes* (type 2) (ATCC 19112) | $1.3 \times 10^8$ | 99.99 | 99.99 |
| *Streptococcus pneumoniae* (type 5) (ATCC 6305) | $6.4 \times 10^7$ | 100 | 100 |
| *Streptococcus mutans* (ATCC 25179) | $6.5 \times 10^6$ | 96.2 | 96.8 |
| *Propionibacterium acnes* (ATCC 6919) | $1.2 \times 10^8$ | 100 | 100 |
| *Enterococcus fecalis* (ATCC 19433) | $3.7 \times 10^7$ | 99.36 | 99.98 |
| *Gardnerella vaginalis* (ATCC 14018) | $5.5 \times 10^7$ | 100 | 100 |
| *Lactobacillus acidophilus* (ATCC 4356) | $5.0 \times 10^5$ | 100 | 100 |

• CPU = colony forming units

Table 6 demonstrates that all gram positive bacteria which cause significant human clinical infections were exquisitely sensitive to the microbicidal action of the GMO/CPC emulsion.

Table 7 shows the percentage of gram negative bacteria killed after a 10 or 30 minute incubation of the GMO/CPC emulsion with between $10^5$ and $10^8$ bacteria. Included in the table are gram negative bacteria which colonize in the oropharynx, such as *Haemophilus influenzae* (capsular type b) and *Neisseria meningitidis* type b; the gastrointestinal tract, such as *Helicobacter pylori*; and the vagina, such as *Neisseria gonorrhoeae.*

TABLE 7

| Gram Negative Bacteria | Innoculum (CFU)• | % Inactivate after a 10 Minute Incubation | % Inactivate after a 30 Minute Incubation |
|---|---|---|---|
| *Escherichia coli* type O18:K1 | $2.1 \times 10^7$ | 97.1 | 96.7 |
| *Escherichia coli* type O18:K- | $3.2 \times 10^7$ | 89.7 | 99.6 |
| *Escherichia coli* J5 (epimerase deficient) | $3 \times 10^7$ | 94 | 85 |
| *Flavobacterium meningosepticum* Group A (ATCC 13253) | $2.1 \times 10^6$ | 0 | 47.6 |
| *Klebsiella pneumonia* type O12K+ | $5 \times 10^7$ | 98.3 | 99.9 |
| *Pseudomonas aeruginosa* type FD-1 | $3.8 \times 10^7$ | 99 | 99 |
| *Pseudomonas aeruginosa* MEP strain 2192 | $8 \times 10^6$ | 99.1 | 97.5 |
| *Haemophilus influenzae* capsular type b (ATCVC 33533) | $1 \times 10^7$ | 99.99 | 99.99 |
| *Neisseria meningitidis type b* (ATCC 13090) | $1.6 \times 10^8$ | 100 | 100 |
| *Neisseria gonorrhoeae* (ATCC 9793) | $1.2 \times 10^6$ | 100 | 100 |
| *Helicobacter pylori* (ATCC 43504) | $3 \times 10^6$ | 100 | 100 |
| *Helicobacter pylori* (ATCC 43629) | $4.7 \times 10^6$ | 100 | 100 |

TABLE 7   (continued)

| Gram Negative Bacteria | Innoculum (CFU) • | % Inactivate after a 10 Minute Incubation | % Inactivate after a 30 Minute Incubation |
|---|---|---|---|
| *Helicobacter pylori* (ATCC 49503) | $2 \times 10^6$ | 100 | 100 |
| *Helicobacter pylori* (ATCC 43579) | $7 \times 10^6$ | 100 | 100 |

Table 7 illustrates that at 30 minutes there is inactivation of at least 85% of the innoculum of all gram negative bacteria tested except for *Flavobacterium meningosepticum.* The encapsulated type b *Haemophilus influenzae,* type b *Neisseria meningitidis, Neisseria gonorrhoeae,* and *Helicobacter pylori* bacteria, which have relatively rough LPS types compared to the other gram negative bacteria, are exquisitely sensitive to the microbicidal activity of the GMO/ CPC emulsion.

Table 8 shows the percentage of the two *Candida* species killed after a 10 or 30 minute incubation of the GMO/CPC emulsion with $10^5$ yeast.

TABLE 8

| Yeast | Innoculum (CPU) | % Inactivate After a 10 Minute Incubation | % Inactivate After a 30 Minute Incubation |
|---|---|---|---|
| *Candida albicans* (clinical blood isolate) | $3.2 \times 10^5$ | 62.5 | 62.5 |
| *Candida tropicalis* (clinical blood isolate) | $5.4 \times 10^5$ | 100 | 100 |

Table 8 demonstrates that significant inactivating of the *Candida albicans* species occurs after only a 10-30 minute incubation with the GMO/CPC emulsion.

## Example 5 - Microbicidal Activity of 10N GMO Emulsions

In this Example, the positively charged 10N GMO emulsions formed in Example 2 and shown in Table 3 were tested for microbicidal activity against two gram positive bacteria, *Staphylococcus aureus* type 8 and *Staphylococcus epidermidis,* and two gram negative bacteria, *E. coli* and *Helicobacter pylori,* as follows:

To lyophilized cultures of bacteria, 0.5 mL of Schaedler's Broth was added and the entire contents of the vial inoculated to a 5% Sheep blood agar plate (TSA II). Cultures were then incubated in an anaerobic jar with Campy GasPAK at 37°C for 72 hours. Colonies from 72 hour TSA 11 plates were then innoculated into Schaedler's broth until an 0.5 McFarland Standard was achieved.

A one ml aliquot of the bacterial culture was then mixed with one ml of oil-in-water emulsion for 10 minutes. Quantitative cultures were then performed in duplicate on each mixture, and a quantitative culture was performed on the original broth culture. Plates containing bacteria were incubated at 37°C for 72 hours in an anaerobic jar with Campy GasPaks and then counted. The percentage of bacteria killed were determined by the following equation and the results are shown in Table 9:

$$\% \text{ Kill} = \frac{(A-B)}{A} \times 100$$

A = The total number of bacteria innoculated

B = The number counted after mixing with an emulsion or liposomes

Table 9 lists the percentages of each bacterium killed after a 10 minute incubation with the 10N GMO/CPC emulsion from Table 3 with between $10^6$ and $10^7$ bacteria. The 10N GMO/CPC emulsion was tested in undiluted form and in several dilutions, as indicated in Table 9.

TABLE 9

| Bacterium | Dilution | Trial #1 | Trial #2 |
|---|---|---|---|
| *Staphylococcus aureus* | Undilute | NT | 100.00% |

TABLE 9 (continued)

| Bacterium | Dilution | Trial #1 | Trial #2 |
|---|---|---|---|
| Type 8<br>8 x 10E7 CFU/mL<br>10 minute incubation | 1 : 10<br>1 : 20<br>1 : 40<br><br>1 : 80 | | 99.99%<br>99.99%<br>99.99%<br><br>96.25% |
| *Staphylococcus*<br>*epidermidis* 977<br>9 X 10E6 CFU/mL<br>10 minute incubation | Undilute<br>1 : 10<br>1 : 20<br>1:40<br><br>1 : 80 | NT | 100.00%<br>100.00%<br>100.00%<br>99.98%<br><br>99.97% |
| *Escherichia coli*<br>Type 02a,2b<br>1 X 10E7 CFU/mL<br>10 minute incubation | Undilute<br>1 : 10<br>1 : 20<br>1 : 40<br><br>1 : 80 | NT | 99.99%<br>99.99%<br>99.98%<br>94.00%<br><br>50.00% |
| *Helicobacter pylori* 43579<br>10 minute incubation<br>Inoculum for Trial #1<br>5X10E6 CFU/ml<br>Inoculum for Trial #2<br>2X10E7 CFU/ml | Undilute<br>1 : 10<br>1 : 20<br><br>1 : 40<br><br>1 : 80 | 100.00%<br>100.00%<br>100.00%<br><br>99.00%<br><br>99.20% | 100.00%<br>100.00%<br>100.00%<br><br>99.00%<br><br>99.00% |
| NT = Not Tested on This Date | | | |

Table 9 shows that at 10 minutes there is a greater than 96% inactivation of each bacterium (except for *E. coli* at high dilutions (i.e., 1 : 40 and 1 : 80)) tested with the 10N GMO/CPC emulsion up to a 1 : 80 dilution. At a dilution of 1 : 10, there is a 99.99-100% inactivation of each bacterium.

## Example 6 - Viricidal Activity of GMO Oil-in-Water Emulsions and Fusogenic Lipid Vesicles Against HIV

In this Example, the viricidal activity of the GMO/CPC emulsion formed in Example 1 and fusogenic lipid vesicles prepared as described below were tested against HIV.

To prepare fusogenic lipid vesicles, 94.5 grams of Polyoxyethylene (POE) 2 Stearyl Ether (Brij 72), 0.78 grams of oleic acid, and 36 grams of cholesterol were heated to melting to form a liquid lipid phase. A portion of that lipid phase, 0.53 ml, was then mixed with 0.47 ml of squalene to form 1.0 ml of a lipid-oil phase. This was blended with an aqueous phase containing 4.0 ml of PBS under shear mixing conditions to form the lipid vesicles, as described in U. S. Patent 4,911,928. "Shear mixing conditions", as used herein, means a shear equivalent to a relative flow of 5-50 m/s through a 1mm orifice. On a volume to volume basis, the mixture contained 4 parts diluent to 1 part lipid-oil mixture, or 11.7% lipid to 8.3% oil to 80% diluent.

The GMO/CPC emulsion and Brij 72 fusogenic lipid vesicles were assayed for viricidal activity against HIV as follows:

A cell suspension containing 20 x $10^6$ C8166 cells, a HTLV-1 transformed T cell line, in 20 ml of RPMI media containing 10% PBS and 50 μg/ml gentamicin was prepared and divided into 0.4 ml aliquots. A series of viral dilutions ($10^{-2}$ to $10^{-6}$) were then made by first mixing 100 microliters of HIV-1$_{Mn}$ 1000 x pelleted virus with either 100 microliters of sterile water or 100 microliters of the GMO/CPC emulsion for 30 minutes at room temperature on a nutator. 1.8 ml of tissue culture medium was then added to each preparation, resulting in a dilution of $10^{-1}$. Serial 10-fold dilutions were then performed from $10^{-2}$ to $10^{-6}$ by taking 150 microliters of the $10^{-1}$ dilution and serially diluting it out in 1.35 ml of RPMI media five more times.

Two 0.4 ml aliquots of each dilution were then added to the aliquots of C8166 cells, prepared as described above. The samples were incubated at 37°C for either 2, 6 or 24 hours, washed 3 times in phosphate buffered saline, resuspended in fresh medium and each dilution plated in four replicate wells. Cells were fed twice weekly. Cells were observed at 7 and 14 days for cytopathic effects and supernatants were collected to measure levels of p24 as an indication of viral proliferation. Tissue culture infectious dose (TCID-50 ($\log_{10}$)) calculations were then performed based on the measured levels of p24. The results were as follows:

For samples incubated for 2 hours with the GMO/CPC emulsion, there was a 4.5 log virus reduction, as compared with water, in the TCID-50 at 7 days and a 4.25 log virus reduction in the TCID-50 at 14 days.

For samples incubated for 6 hours with the GMO/CPC emulsion, there was a 4.5 log virus reduction, as compared with water, in the TCID-50 at 7 days and a 5 log virus reduction, as compared with water, in the TCID-50 at 14 days.

For samples incubated for 24 hours with the GMO/CPC emulsion, there was a 4.0 log virus reduction, as compared with water, in the TCID-50 at 7 and 14 days; there was a 3.75 and 4.25 log reduction, as compared with Phosphate Buffered Saline, in the TCID-50 at 7 and 14 days, respectively; and there was a 4.0 and 4.5 log reduction, as compared with fusogenic novasomes containing POE 2 Stearyl Ether, in the TCID-50 at 7 and 14 days, respectively.

Overall, this Example demonstrates that GMO/CPC emulsions inactivate 4.0 to 5.0 logs of HIV-$1_{Mn}$ infectivity in C8166 tissue culture assays. These experiments, which can be performed using any enveloped virus, demonstrate the effectiveness of these materials to inactivate viruses such as HIV-$1_{Mn}$.

### Example 7 - Stability of GMO Oil-in-Water Emulsions

In this Example, the GMO/CPC emulsion formed in Example 1 was tested for stability in the presence of heat, acid and base. Table 10 shows the effect of boiling for one hour on breakdown or aggregation of the GMO/CPC emulsion, shows the effects of mixing equal volumes of IN Nitric acid and GMO/CPC emulsion for two hours on breakdown or aggregation of the GMO/CPC emulsion, and the effects of mixing equal volumes of IN Sodium hydroxide and GMO/CPC emulsion for two hours on breakdown or aggregation of the GMO/CPC emulsion.

TABLE 10

| Chemical Components of Emulsion | Intervention | Mean Coulter Size in Microns | Mean Coulter Range in Microns |
|---|---|---|---|
| GMO/CPC | No boiling | 1.008 | 0.720-1.337 |
| GMO/CPC | Boiling 1 hour | 1.167 | 0.654-1.517 |
| GMO/CPC | No acid treatment | 1.008 | 0.720-1.337 |
| GMO/CPC | 1N HNO$_3$ for 2 hours | 1.062 | 0.675-1.569 |
| GMO/CPC | No base treatment | 1.008 | 0.720-1.337 |
| GMO/CPC | 1N NaOH for 2 hours | 0.804 | 0.658-0.969 |

Table 10 shows that: (a) boiling for 1 hour does not significantly alter the breakdown or size of the emulsion; (b) 1N Nitric acid exposure for 2 hours does not significantly alter the size or aggregate profile of the CMO/CPC emulsion; and (c) 1N Sodium hydroxide exposure for 2 hours causes a 20% decrease in the mean size of the emulsion without disrupting the emulsion or causing aggregation.

From the above-described Examples 1-7, it is evident that the antimicrobial oil-in-water emulsions of the present invention have significant microbicidal activity against a wide variety of viruses, bacteria and yeast, even at extremely low concentrations of cationic halogen-containing compound, such as CPC. Furthermore, the emulsions of the invention are stable in the presence of heat, acid, and base, making them very suitable for pharmaceutical administration, whether topical, oral or systemic.

Those skilled in the art will recognize, or be able to ascertain using no more than routine experimentation, many equivalents to the specific embodiments of the invention described herein. Such equivalents are intended to be encompassed by the following claims.

### Claims

1.　An antimicrobial oil-in-water emulsion comprising positively charged droplets of an oily discontinuous phase dispersed in a continuous aqueous phase, the emulsion comprising:

> a. an oil;
> b. glycerol monooleate as the primary emulsifier; and
> c. a cationic halogen-containing compound having a $C_{12}$-$C_{16}$ chain selected from cetylpyridinium chloride, cetylpyridinium bromide, cetyltrimethylammonium bromide, cetyltrimethylammonium chloride, cetyldimethylethylammonium bromide, cetylbenzyldimethylammonium chloride, cetyltributylphosphonium bromide, dodecyltrimethylammonium bromide, and tetradecyltrimethylammonium bromide.

2. An antimicrobial emulsion according to Claim 1, wherein the oil is selected from soybean oil, squalene oil, sesame oil, olive oil, canola oil, corn oil, rapeseed oil, safflower oil, sunflower oil, fish oils, avocado oil, flavour oils, petrolatum, triglyceride oils and fats, water insoluble vitamins, and mixtures thereof; and/or the cationic halogen- containing compound is cetylpyridinium chloride.

3. An antimicrobial emulsion according to Claim 1 or 2, wherein the emulsion further comprises at least ore sterol selected from cholesterol, cholesterol derivatives, hydrocortisone, phytosterol, and mixtures thereof.

4. An antimicrobial emulsion according to any of Claims 1 to 3, wherein at least a portion of the emulsion is in the form of a positively charged lipid structure selected from unilamellar, multilamellar, and paucilamellar lipid vesicles, micelles, and lamellar phases.

5. An antimicrobial preparation suitable for pharmaceutical administration comprising an antimicrobial emulsion according to any of Claims 1 to 4 and a pharmaceutically acceptable carrier.

6. An antimicrobial preparation according to Claim 5 suitable for topical application, for inhibiting the growth of an infectious pathogen.

7. An antimicrobial preparation according to Claim 6, wherein said topical application comprises oral application; application to a mucous membrane via inhalation; or vaginal application.

8. An antimicrobial preparation according to Claim 6 or 7, wherein the infectious pathogen is a bacterium, fungus or virus.

9. An antimicrobial preparation according to Claim 8, wherein the virus is an enveloped virus.

10. An antimicrobial preparation according to Claim 9, wherein the enveloped virus is selected from *Herpesviridae, Togaviridae, Flaviviridae, Coronaviridae, Rhabdoviridae, Filoviridae, Paramyxoviridae, Orthomyxovirldae, Bunyaviridae, Arenaviridae, Retroviridae,* and *Hepadnavirida,* preferably HIV.

11. A method of inactivating an infectious pathogen in vitro comprising the step of contacting the pathogen with an antimicrobial oil-in-water emulsion according to any of Claims 1 to 4.

12. A method according to Claim 11, wherein the pathogen is a virus, especially HIV.

**Patentansprüche**

1. Antimikrobielle Öl-in-Wasser-Emulsion, die positiv geladene Tröpfchen einer öligen diskontinuierlichen Phase dispergiert in einer kontinuierlichen wässrigen Phase umfasst, wobei die Emulsion Folgendes umfasst:

> a. Ein Öl;
> b. Glycerinmonooleat als das primäre Emulgiermittel und
> c. eine kationische, Halogen enthaltende Verbindung mit einer $C_{12}$-$C_{16}$-Kette, ausgewählt aus: Cetylpyridiniumchlorid, Cetylpyridiniumbromid, Cetyltrimethylammoniumbromid, Cetyltrimethylammoniumchlorid, Cetyldimethylethylammoniumbromid, Cetylbenzyldimethylammoniumchlorid, Cetyltributylphosphoniumbromid, Dodecyltrimethylammoniumbromid und Tetradecyltrimethylammoniumbromid.

2. Antimikrobielle Emulsion nach Anspruch 1, worin das Öl ausgewählt wird aus: Sojabohnenöl, Squalen-Öl, Sesamöl, Olivenöl, Canolaöl, Maisöl, Rapsöl, Safloröl, Sonnenbumenöl, Fischölen, Avocadoöl, Aromaölen, Petrolatum, Triglyceridölen und -fetten, wasserunlöslichen Vitaminen und Gemischen davon; und/oder die kationische, Halogen enthaltende Verbindung Cetylpyridiniumchlorid ist.

3.  Antimikrobielle Emulsion nach Anspruch 1 oder 2, worin die Emulsion ferner mindestens ein Sterin umfasst, ausgewählt aus Cholesterin, Cholesterinderivaten, Hydrocortison, Phytosterin und Gemischen davon.

4.  Antimikrobielle Emulsion nach einem der Ansprüche 1 bis 3, worin mindestens ein Anteil der Emulsion in der Form einer positiv geladenen Lipidstruktur vorliegt, ausgewählt aus unilamellaren, multilamellaren und paucilamellaren Lipidvesikeln, Micellen und lamellaren Phasen.

5.  Antimikrobielle Zubereitung geeignet zur pharmazeutischen Verabreichung, die eine antimikrobielle Emulsion nach einem der Ansprüche 1 bis 4 und einen pharmazeutisch zulässigen Träger umfasst.

6.  Antimikrobielle Zubereitung nach Anspruch 5, geeignet zur topischen Applikation, zum Inhibieren des Wachstums eines infektiösen Pathogens.

7.  Antimikrobielle Zubereitung nach Anspruch 6, worin genannte topische Applikation orale Applikation; Applikation über Inhalation auf eine Schleimhaut; oder vaginale Applikation umfasst.

8.  Antimikrobielle Zubereitung nach Anspruch 6 oder 7, worin das infektiöse Pathogen ein Bacterium, Fungus oder Virus ist.

9.  Antimikrobielle Zubereitung nach Anspruch 8, worin das Virus ein in einer Umhüllung eingeschlossenes Virus ist.

10. Antimikrobielle Zubereitung nach Anspruch 9, worin das in eine Umhüllung eingeschlossene Virus ausgewählt wird aus: *Herpesviridae, Togaviridae, Flaviviridae, Coronaviridae, Rhabdoviridae, Filoviridae, Paramyxoviridae, Orthomyxoviridae, Bunyaviridae, Arenaviridae, Retroviridae* und *Hepadnaviridae,* bevorzugt HIV.

11. Verfahren zur Inaktivierung eines infektiösen Pathogens in vitro, das den Schritt des Kontaktierens des Pathogens mit einer antimikrobiellen Öl-in-Wasser-Emulsion nach einem der Ansprüche 1 bis 4 umfasst.

12. Verfahren nach Anspruch 11, worin das Pathogen ein Virus, besonders HIV ist.

**Revendications**

1.  Emulsion huile-dans-eau antimicrobienne comprenant des gouttelettes chargées positivement d'une phase discontinue huileuse dispersées dans une phase aqueuse continue, l'émulsion comprenant :

    a. une huile ;
    b. du monooléate de glycérol comme agent émulsifiant primaire ; et
    c. un composé cationique halogéné ayant une chaîne en $C_{12}$-$C_{16}$ choisi parmi le chlorure de cétylpyridinium, le bromure de cétylpyridinium, le bromure de cétyltriméthylammonium, le chlorure de cétyltriméthylammonium, le bromure de cétyldiméthyléthylammonium, le chlorure de cétylbenzyldiméthylammonium, le bromure de cétyltributylphosphonium, le bromure de dodécyltriméthylammonium et le bromure de tétradécyltriméthylammonium.

2.  Emulsion antimicrobienne selon la revendication 1, dans laquelle l'huile est choisie parmi l'huile de soja, l'huile de squalène, l'huile de sésame, l'huile d'olive, l'huile de canola, l'huile de maïs, l'huile de colza, l'huile de carthame, l'huile de tournesol, les huiles de poisson, l'huile d'avocat, les huiles aromatiques, le pétrolatum, les huiles et les matières grasses de triglycérides, les vitamines insolubles dans l'eau, et des mélanges de ceux-ci ; et/ou le composé cationique halogéné est le chlorure de cétylpyridinium.

3.  Emulsion antimicrobienne selon la revendication 1 ou 2, dans laquelle l'émulsion comprend en outre au moins un stérol choisi parmi le cholestérol, les dérivés du cholestérol, l'hydrocortisone, le phytostérol et des mélanges de ceux-ci.

**4.** Emulsion antimicrobienne selon l'une quelconque des revendications 1 à 3, dans laquelle au moins une portion de l'émulsion est sous la forme d'une structure lipidique chargée positivement, choisie parmi les vésicules lipidiques unilamellaires, multilamellaires et paucilamellaires, les micelles et les phases lamellaires.

**5.** Préparation antimicrobienne convenable pour une administration pharmaceutique, comprenant une émulsion antimicrobienne selon l'une quelconque des revendications 1 à 4 et un véhicule pharmaceutiquement acceptable.

**6.** Préparation antimicrobienne selon la revendication 5, convenable pour une application topique, pour l'inhibition de la croissance d'un pathogène infectieux.

**7.** Préparation antimicrobienne selon la revendication 6, dans laquelle ladite application topique comprend l'application orale ; l'application à une muqueuse par inhalation ; ou l'application vaginale.

**8.** Préparation antimicrobienne selon la revendication 6 ou 7, dans laquelle le pathogène infectieux est une bactérie, un fongus ou un virus.

**9.** Préparation antimicrobienne selon la revendication 8, dans laquelle le virus est un virus enveloppé.

**10.** Préparation antimicrobienne selon la revendication 9, dans laquelle le virus enveloppé est choisi parmi Herpesviridae, Togaviridae, Flaviviridae, Coronaviridae, Rhabdoviridae, Filoviridae, Paramyxoviridae, Orthomyxoviridae, Bunyaviridae, Arenaviridae, Retroviridae et Hepadnaviridae, préférablement le VIH.

**11.** Méthode d'inactivation d'un pathogène infectieux in vitro, comprenant l'étape de mise en contact du pathogène avec une émulsion huile-dans-eau antimicrobienne selon l'une quelconque des revendications 1 à 4.

**12.** Méthode selon la revendication 11, dans laquelle le pathogène est un virus, notamment le VIH.